(19) Europäisches Patentamt
European Patent Office
Office européen des brevets

(11) EP 2 380 439 A1

# (12) EUROPEAN PATENT APPLICATION

published in accordance with Art. 153(4) EPC

(43) Date of publication:
**26.10.2011 Bulletin 2011/43**

(21) Application number: **09833519.3**

(22) Date of filing: **21.12.2009**

(51) Int Cl.:
***A01N 43/90*** *(2006.01)* ***A01N 43/40*** *(2006.01)*
***A01N 47/02*** *(2006.01)* ***A01N 47/40*** *(2006.01)*
***A01N 51/00*** *(2006.01)* ***A01P 3/00*** *(2006.01)*
***A01P 7/02*** *(2006.01)* ***A01P 7/04*** *(2006.01)*

(86) International application number:
**PCT/JP2009/071255**

(87) International publication number:
**WO 2010/071218 (24.06.2010 Gazette 2010/25)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.12.2008 JP 2008323739**

(71) Applicant: **Meiji Seika Pharma Co., Ltd.**
**Tokyo 104-8002 (JP)**

(72) Inventors:
- **HORIKOSHI Ryo**
  **Yokohama-shi**
  **Kanagawa 222-8567 (JP)**
- **NOMURA Masahiro**
  **Yokohama-shi**
  **Kanagawa 222-8567 (JP)**
- **NAKAMURA Satoshi**
  **Yokohama-shi**
  **Kanagawa 222-8567 (JP)**
- **HAYASHIMOTO Mizuki**
  **Yokohama-shi**
  **Kanagawa 222-8567 (JP)**
- **TSUCHIDA Mariko**
  **Yokohama-shi**
  **Kanagawa 222-8567 (JP)**
- **OYAMA Kazuhiko**
  **Yokohama-shi**
  **Kanagawa 222-8567 (JP)**
- **MITOMI Masaaki**
  **Yokohama-shi**
  **Kanagawa 222-8567 (JP)**

(74) Representative: **Tetaz, Franck Claude Edouard et al**
**Cabinet Regimbeau**
**139, rue Vendôme**
**69477 Lyon Cedex 06 (FR)**

## (54) COMPOSITION TO CONTROL HARMFUL ORGANISMS THAT CONTAINS 16-KETO ASPERGILLIMIDE

(57) Disclosed is a composition for use in controlling harmful organisms, comprising as active ingredients at least one of 16-keto aspergillimide, its enantiomers, their mixture, or their acid addition salts and at least one of other harmful organism control agents. The present invention provides a composition for use in controlling harmful organisms that has excellent insecticidal activity or penetrative transferable insecticidal activity against a wide range of harmful organisms, particularly agricultural and horticultural insect pests.

EP 2 380 439 A1

## Description

[CROSS-REFERENCE TO RELATED APPLICATIONS]

The application is based upon and claims the benefit of priority from the prior Japanese Patent Application No. 323739/2008, filed on December 19, 2008; the entire contents of which are incorporated herein by reference.

[BACKGROUND OF THE INVENTION]

### Field of Invention

The present invention relates to a composition for use in controlling harmful organisms that comprises 16-keto aspergillimide (PF1378 substance) and at least one of other harmful organism control agents.

### Background Art

A number of harmful organism control agents have hitherto been known. However, agents that can be effectively and safely used have been required from the viewpoints of drug resistance and safety. Further, in an agricultural and horticultural field, a method in which a chemical agent is applied to seeds or plants in a sowing time, nursery period, or transplanting is widely used as a method for use in controlling pests of agricultural crops contemplated for work saving in farmers. In this case, a penetrative transferable agent that is absorbed from seeds or the root of plants and is penetrated into the plants to exhibit control effect is generally used. The penetrative transferable agent, also known as a systemic insecticide, refers to a chemical agent that is penetrated and transferred into a plant and is sucked or eaten by pests, whereby the pests die by poisoning (see New edition "Noyaku no Kagaku (Agrochemical Science)" (Kyohei Yamashita et al., Buneido Publishing Co., Ltd.), p. 14 (Non-patent Document 1)).
PF1378 substance, that is, 16-keto aspergillimide, is a compound having the following structure that is reported as a metabolitic product produced by an organism belonging to Aspergillus and is known as an anthelmintically active substance against larvae at the third instar born of Haemonchus contortus which is a parasitic nematode in a digestive tract of mammals (The Journal of Antibiotics, 1997, 50(10), 840-846 (Non-patent Document 2)). However, there is no report about the activity of this substance against agricultural pests.

[Chemical formula 1]

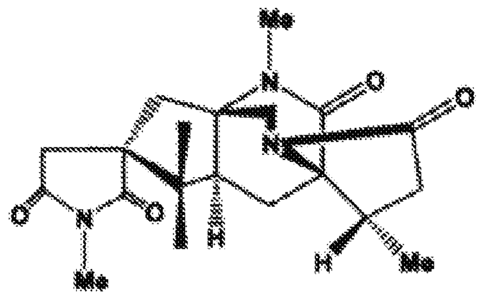

Further, Tetrahedron Letters, 1997, 38 (32), 5655-5658 (Non-Patent Document 3), Bioscience, Biotechnology and Biochemistry, 2000, 64 (1), 111-115 (Non-Patent Document 4), and Japanese Patent Application Laid-Open Publication No. 245383/1998 (Patent Document 1) report that asperparalines which are substances analogous to 16-keto aspergillimide have stupefacient activity against silkworm ( Bombyx mori) or insecticidal activity against beet armyworm (Spodoptera exigua), brown planthopper (Nilaparvata lugens (Stal)) and cockroach . They, however, do not report at all that the 16-keto aspergillimide has insecticidal activity, systemic insecticidal activity, and the effect of a composition comprising 16-keto aspergillimide and other harmful organism control agents.
Up to now, a number of harmful organism control agents are reported. For all the harmful organism control agents, the presence of insect pest species having drug resistance and hardly controllable insect pest species have been confirmed. Accordingly, it can be said that the development of a novel harmful organism control agent is still desired. Further, it can be said from the viewpoints of ecological sensitivity and influence on organism other than target organisms that control with small dosage is desired.

[PRIOR ART REFERENCES]

[PATENT DOCUMENTS]

[Patent Document 1] Japanese Patent Application Laid-Open Publication No. 245383/1998

[NON-PATENT DOCUMENTS]

[Non-patent Document 1] New edition "Noyaku no Kagaku (Agrochemical Science)" (Kyohei Yamashita et al., Buneido Publishing Co., Ltd.), p. 14
[Non-patent Document 2] The Journal of Antibiotics, 1997, 50(10), 840-846
[Non-patent Document 3]Tetrahedron Letters, 1997, 38(32), 5655-5658
[Non-patent Document 4]Bioscience, Biotechnology and Biochemistry, 2000, 64(1), 111-115

[SUMMARY OF THE INVENTION]

The present inventors have now found that a composition comprising at least one of 16-keto aspergillimide, its enantiomers, their mixture, or their acid addition salts and at least one of other harmful organism control agents has excellent insecticidal activity or systemic insecticidal activity against a wide range of harmful organisms, particularly agricultural and horticultural pests. The present invention has been made based on such finding.
Accordingly, an object of the present invention is to provide a composition for use in controlling harmful organisms that has excellent insecticidal activity or systemic insecticidal activity against a wide range of harmful organisms, particularly agricultural and horticultural pests.
According to one aspect of the present invention, there is provided a composition for use in controlling harmful organisms, the composition comprising as active ingredients at least one of 16-keto aspergillimide, its enantiomers, their mixture, or their acid addition salts and at least one of other harmful organism control agents.
According to another aspect of the present invention, there is provided a combination comprising as active ingredients at least one of 16-keto aspergillimide, its enantiomers, their mixture, or their acid addition salts and at least one of other harmful organism control agents.
According to still another aspect of the present invention, there is provided a method for protecting useful plants or animals from harmful organisms, the method comprising applying an effective amount of the compositon for use in controlling harmful organisms to an object, wherein the object is a harmful organism, a useful plant, a seed of a useful plant, a soil, a cultivation material, or an animal.
According to a further aspect of the present invention, there is provided use of the composition for use in controlling harmful organisms for the protection of useful plants or animals from harmful organisms.

[DETAILED DESCRIPTION OF THE INVENTION]

<u>16-Keto aspergillimide</u>

16-Keto aspergillimide can be obtained from a culture of a 16-keto aspergillimide producing microorganism according to a method described, for example, in The Journal of Antibiotics, 1997, 50 (10), 840-846 (non-patent document 2).
According to the present invention, not only 16-keto aspergillimide but also its enantiomers and their mixture can be utilized.
Further, in the present invention, acid addition salts of 16-keto aspergillimide, its enantiomers, or their mixture (preferably agriculturally and zootechnically acceptable acid addition salts) may also be utilized, and examples thereof include acid addition salts such as hydrochloride, nitrate, sulfate, phosphate or acetate.

<u>Composition for use in controlling harmful organisms</u>

16-Keto aspergillimide as such has excellent control effect against harmful organisms. It has been found that a mixture of 16-keto aspergillimide with other organism control agent(s) exhibits better control effect than the control effect attained by the sole use of the 16-keto aspergillimide or the other organism control agent(s). Accordingly, according

to the present invention, there is provided a composition for use in controlling harmful organisms, the composition comprising at least one of 16-keto aspergillimide, its enantiomers, their mixture, or their acid addition salts and at least one of other harmful organism control agent.

According to another preferred aspect of the present invention, there is provided a composition for use in controlling harmful organisms, the composition comprising at least one of 16-keto aspergillimide, its enantiomers, their mixture, or their acid addition salts and at least one of other agricultural and horticultural insecticides and/or fungicides.

According to still another preferred aspect of the present invention, there is provided a composition for use in controlling harmful organisms, the composition comprising at least one of 16-keto aspergillimide, its enantiomers, or their mixture and at least one of other a parasite control agents for animals.

According to another preferred aspect of the present invention, there is provided a composition for use in controlling harmful organisms, the composition comprising at least one of 16-keto aspergillimide, its enantiomers, or their mixture and at least one of other ectoparasite control agents for animals.

Insect pest species against which the composition for use in controlling harmful organisms, comprising at least one of 16-keto aspergillimide, its enantiomers, their mixture, or their acid addition salts and at least one of other harmful organism control agents exhibits insect pesticidal effect include, but are not limited to, agricultural and horticultural insect pests, for example, lepidopteran insect pests, for example, Spodoptera litura, Mamestra brassicae, Pseudaletia separata, Pieris rapae, Plutella xylostella, Spodoptera exigua, Chilo suppressalis, Cnaphalocrocis medinalis, Tortricidae, Carposinidae, Lyonetiidae, Lymantriidae, Agrotis spp., Helicoverpa spp., and Heliothis spp.; hemipteran insect pests, for example, Aphididae, Adelgidae or Phylloxeridae such as Myzus persicae, Aphis gossypii, Aphis fabae, corn leaf aphid (Rhopalosiphum maidis), Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola, Rosy apple aphid, Eriosoma lanigerum, Toxoptera aurantii, and Toxoptera citricidus; Cicadellidae such as Nephotettix cincticeps, Delphacidae such as Laodelphax striatellus, Nilaparvata lugens, and Sogatella furcifera; heteropteran insect pests such as Eysarcoris ventralis, Nezara viridula, and Trigonotylus caelestialium; Aleyrodidae such as Bemisia tabaci, and Trialeurodes vaporariorum; Coccidea (Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, or Cerococcidae), such as Pseudococcus comstocki and Planococcus citri; Coleopteran insect pests, for example, Lissorhoptrus oryzophilus, Callosobruchuys chienensis, Tenebrio molitor, Diabrotica virgifera virgifera, Diabrotica undecimpunctata howardi, Anomala cuprea, Anomala rufocuprea, Phyllotreta striolata, Aulacophora femoralis, Leptinotarsa decemlineata, Oulema oryzae, Bostrychidae, and Cerambycidae; Acari, for example, Tetranychus urticae, Tetranychus kanzawai, and Panonychus citri; Hymenopteran insect pests, for example, Symphyta; Orthopteran insect pests, for example, Acrididae; Dipteran insect pests, for example, Muscidae and Agromyzidae; Thysanopteran insect pests, for example, Thrips palmi and Frankliniella occidentalis; Plant Parasitic Nematodes, for example, Meloidogynidae, Pratylenchidae, Aphelenchoides besseyi and Bursaphelenchus xylophilus; and zooparasites, for example, Siphonaptera, for example, Ctenocephalides felis and Pulex irritans, Anoplura, for example, Pediculus humanus and Pediculus pubis, zoobiotic mites or ticks such as Boophilus microplus., Haemaphysalis longicornis, Rhipicephalus sanguineus, Haemaphysalis flava, Sarcoptes scabiei, Dermanyssus gallinae, Ornithonyssus sylviarum, Ornithonyssus bacoti, and Leptotrombidium, horseflies, flies, for example, Lucilia spp., mosquitoes, for example, Stegomyia albopicta and Culex pipiens pallens, Simuliidae, Ceratopogonidae, Trematoda, Acanthocephala, Cestoda, Nematoda, Sarcomastigophora and Ciliata, and Sporozoa. More preferred are Hemipteran insect pests, Thysanopteran insect pests, Dipteran insect pests, Coleoptera insect pests, zoobiotic Aphaniptera, and zoobiotic mites or ticks. Still more preferred are Hemipteran insect pests and zoobiotic mites or ticks.

In the present specification, harmful organism control agents admixable into at least one of 16-keto aspergillimide, its enantiomers, their mixture, or their acid addition salts include insecticides, fungicides, miticides or tickicides, herbicides, plant growth-regulating agents, and parasite control agents for animals. Specific agents include those described, for example, in The Pesticide Manual, 13th edition, published by The British Crop Protection Council; and SHIBUYA INDEX, the 13th edition, 2008, published by SHIBUYA INDEX RESEARCH GROUP. Preferred other harmful organism control agents admixable into at least one of 16-keto aspergillimide, its enantiomers, their mixture, or their acid addition salts include insecticides and/or bactericides and/or parasite control agents for animals. More preferred are insecticides and/or parasite control agents for animals. Bactericides are additionally admixable into insecticides and/or parasite control agents for animals.

Preferred examples of harmful organism control agents admixable into at least one of 16-keto aspergillimide, its enantiomers, their mixture, or their acid addition salts include organic phosphoric ester compounds, carbamate compounds, nereistoxin derivatives, organochlorine compounds, pyrethroid compounds, benzoyl urea compounds, juvenile hormone analogies , molting hormone analogies, neonicotinoid compounds, sodium channel blockers for nerve cells, insecticidal macrocyclic lactones, γ-aminobutyric acid (GABA) antagonists, ryanodine receptor agonistic compounds, insecticidal ureas, BT agents, entomopathogenic viral agents, polyether antibiotics, thiamine antagonists, and sulfa drugs/folic acid antagonist compounding agents. More specific examples thereof include acephate, dichlorvos,

EPN, fenitothion, fenamifos, prothiofos, profenofos, pyraclofos, chlorpyrifos-methyl, haloxon, coumaphos, malathion, dimpylate, naled, tetradifon, diazinon, methomyl, thiodicarb, aldicarb, oxamyl, propoxur, carbaryl, fenobucarb, ethiofencarb, fenothiocarb, pirimicarb, carbofuran, benfuracarb, cartap, thiocyclam, dicofol, tetradifon, permethrin, tefluthrin, cypermethrin, deltamethrin, cyhalothrin, fenvalerate, fluvalinate, ethofenprox, silafluofen, diflubenzuron, teflubenzuron, flufenoxuron, chlorfluazuron, methoprene, chromafenozide, buprofezin, hexythiazox, amitraz, chlordimeform, pyridaben, fenpyroxymate, pyrimidifen, tebufenpyrad, fluacrypyrim, acequinocyl, cyflumetofen, flubendiamide, ethiprole, fipronil, ethoxazole, imidacloprid, clothianidin, thiamethoxam, thiacloprid, sulfoxaflor, pymetrozine, bifenazate, spirodiclofen, spiromesifen, flonicamid, chlorfenapyr, pyriproxyfene, indoxacarb, pyridalyl, spinosad, avermectin, milbemycin, milbemycin oxime, maduramycin, BT agents, entomopathogenic viral agents, emamectinbenzoate, spinetoram, pyrifluquinazon, chlorantraniliprole, cyenopyrafen, spirotetramat, lepimectin, metaflumizone, pyrafluprole, pyriprole, dimefluthrin, fenazaflor, hydramethylnon, triazamate, ivermectin, selamectin, moxidectin, doramectin, eprinomectin, dinotefuran, nitenpyram, acetamiprid, allethrin, d.d-T allethrin, dl.d-T80 allethrin, pyrethrins, phenothrin, flumethrin, cyfluthrin, d.d-T80 prarethrin, phthalthrin, transfluthrine, resmethrin, cyphenothrin, pyrethrum extract, synepirin 222, synepirin 500, pyriproxyfene, lufenuron, deet, trichlofon, tetrachlorvinphos, bromofenofos, cythioate, metoxadiazon, cyromazine, triflumuron, star anise oil, triclabendazole, flubendazole, fenbendazole, parbendazole, tiabendazole, antimony sodium gluconate, levamisole hydrochloride, bithionol, dichlorofen, bromopropylate, piperonylbutoxide, phenothiazine, piperazine carbon bisulfide, piperazine phosphate, piperazine adipate, piperazine citrate, melarsomine dihydrochloride, metyridine, santonin, pyrantel pamoate, pyrantel, morantel, praziquantel, febantel, emodepside, hygtomycin B, destomycin A, clorsulon, nitroxynil, diamfenethide, antimony sodium gluconate, levamisole hydrochloride, melrsonyl, dithiazanine iodide, stibophen, disophenol, dietylcarbamazine, diminazene, acrinamine, metronidazole, santonin, bunamidine, arecoline, compounds represented by formula (I) or agriculturally and horticulturally acceptable acid addition salts thereof:

[Chemical formula 2]

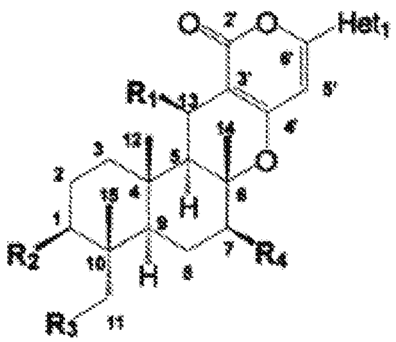

(I)

wherein $Het_1$ represents 3-pyridyl; $R_1$ represents hydroxyl; $R_2$ and $R_3$ represent cyclopropylcarbonyloxy; and $R_4$ represents a hydrogen atom or hydroxyl.

Examples of preferred other insecticides admixable into at least one of 16-keto aspergillimide, its enantiomers, their mixtures, or their acid addition salts include organic phosphoric ester compounds, carbamate compounds, nereistoxin derivatives, organochlorine compounds, pyrethroid compounds, benzoyl urea compounds, juvenile hormone analogies , molting hormone analogies, neonicotinoid compounds, sodium channel blockers for nerve cells, insecticidal macrocyclic lactones, γ-aminobutyric acid (GABA) antagonists, ryanodine receptor agonistic compounds, insecticidal ureas, BT agents, and entomopathogenic viral agents, and specific examples of more preferred insecticides admixable into at least one of 16-keto aspergillimide, its enantiomers, their mixtures, or their acid addition salts include acephate, dichlorvos, EPN, fenitothion, fenamifos, prothiofos, profenofos, pyraclofos, chlorpyrifos-methyl, diazinon, methomyl, thiodicarb, aldicarb, oxamyl, propoxur, carbaryl, fenobucarb, ethiofencarb, fenothiocarb, pirimicarb, carbofuran, benfuracarb, cartap, thiocyclam, dicofol, tetradifon, permethrin, tefluthrin, cypermethrin, deltamethrin, cyhalothrin, fenvalerate, fluvalinate, ethofenprox, silafluofen, diflubenzuron, teflubenzuron, flufenoxuron, chlorfluazuron, methoprene, chromafenozide, buprofezin, hexythiazox, amitraz, chlordimeform, pyridaben, fenpyroxymate, pyrimidifen, tebufenpyrad, fluacrypyrim, acequinocyl, cyflumetofen, flubendiamide, ethiprole, fipronil, ethoxazole, imidacloprid, chlothianidin, thiamethoxam, thiacloprid, sulfoxaflor, pymetrozine, bifenazate, spirodiclofen, spiromesifen, flonicamid, chlorfenapyr, pyriproxyfene, indoxacarb, pyridalyl, spinosad, avermectin, milbemycin, BT agents, entomopathogenic viral agents, emamectinbenzoate, spinetoram, pyrifluquinazon, chlorantraniliprole, cyenopyrafen, spirotetramat, lepimectin, metaflumizone, pyrafluprole, pyriprole, dimefluthrin, fenazaflor, hydramethylnon, triazamate, ivermectin, selamectin, moxidectin, doramectin, eprinomectin, dinotefuran, nitenpyram, acetamiprid, and compounds represented by formula (I) or agriculturally and horticulturally acceptable acid addition salts thereof.

Examples of more preferred other insecticides admixable into at least one of 16-keto aspergillimide, its enantiomers, their mixtures, or their acid addition salts include imidacloprid, nitenpyram, clothianidin, acetamiprid, dinotefuran, thiacloprid, thiamethoxam, sulfoxaflor, fipronil, ivermectin, or compounds of formula (I) or agriculturally and horticulturally acceptable acid addition salts thereof. Still more preferred are chlothianidin, imidacloprid, fipronil, dinotefuran, or compounds of formula (I) or agriculturally and horticulturally acceptable acid addition salts thereof.

Examples of other bactericides admixable into at least one of 16-keto aspergillimide, its enantiomers, their mixtures, or their acid addition salts include strobilrin compounds such as azoxystrobin, kresoxym-methyl, trifloxystrobin, orysastrobin, picoxystrobin, and fuoxastrobin, anilinopyrimidine compounds such as mepanipyrim, pyrimethanil, and cyprodinil, azole compounds such as triadimefon, bitertanol, triflumizole, etaconazole, propiconazole, penconazole, flusilazole, myclobutanil, cyproconazole, tebuconazole, hexaconazole, prochloraz, and simeconazole, quinoxaline compounds such as quinomethionate, dithiocarbamate compounds such as maneb, zineb, mancozeb, polycarbamate, and propineb, phenyl carbamate compounds such as diethofencarb, organochlorine compounds such as chlorothalonil, and quintozene, benzimidazole compounds such as benomyl, thiophanate-methyl, and carbendazole, phenylamides such as metalaxyl, oxadixyl, ofurase, benalaxyl, furalaxyl, and cyprofuram, sulfenic acid compounds such as dichlofluanid, copper compounds such as copper hydroxide, and oxine-copper, isoxazole compounds such as hydroxyisoxazole, organophosphorus compounds such as fosetyl-aluminium and tolclofos-methyl, N-halogenothioalkyl compounds such as captan, captafol, and folpet, dicarboxyimide compounds such as procymidone, iprodione, and vinchlozolin, acid amide compounds such as thifluzamide and furametpyr, benzanilide compounds such as flutolanil and mepronil, morpholine compounds such as fenpropimorph and dimethomorph, organotin compounds such as fenthin hydroxide and fenthinacetate, cyanopyrrole compounds such as fludioxonil and fenpiclonil, other fthalides, probenazole, acibenzolar-S-methyl, tiadinil, isotianil, carpropamid, diclocymet, fenoxanil, tricyclazole, pyroquilon, tebufloquin, ferimzone, fluazinam, cymoxanil, triforine, pyrifenox, fenarimol, fenpropidin, pencycuron, cyazofamid, cyflufenamid, boscalid, penthiopyrad, proquinazid, quinoxyfen, famoxadone, fenamidone, iprovalicarb, benthiavalicarb-isopropyl, fluopicolide, pyribencarb, kasugamycin, or validamycin.

Examples of more preferred other fungicides admixable into at least one of 16-keto aspergillimide, its enantiomers, their mixtures, or their acid addition salts include orysastrobin, thifluzamide, furametpyr, fthalide, probenazole, acibenzolar-S-methyl, tiadinil, isotianil, carpropamid, diclocymet, fenoxanil, tricyclazole, pyroquilon, tebufloquin and ferimzone. More preferred examples thereof include probenazole.

Examples of other parasite control agents for animals admixable into at least one of 16-keto aspergillimide, its enantiomers, their mixtures, or their acid addition salts include pyrethroid compounds, neonicotinoid compounds, avermectin compounds, macrolide compounds, phenylpyrazole compounds, phenylpyrrole compounds, organophosphorus compounds, carbamate compounds, nereistoxin derivatives, organochlorine compounds, benzoyl urea compounds, juvenile hormone analogies, molting hormone analogies, spinosyn compounds, cyclodepsipeptide compounds, sodium channel blockers for nerve cells, insecticidal macrocyclic lactones, $\gamma$-aminobutyric acid (GABA) antagonists, ryanodine receptor agonistic compounds, polyether antibiotics, thiamine antagonists and sulfa drugs/folic acid antagonist compounding agents. More specific preferred examples thereof include ivermectin, selamectin, moxidectin, doramectin, eprinomectin, milbemycin oxime, maduramycin, imidacloprid, dinotefuran, nitenpyram, acetamiprid, thiacloprid, thiamethoxam, clothianidin, sulfoxaflor, fipronil, ethiprole, pyriprole, allethrin, d.d-T allethrin, dl.d-T80 allethrin, pyrethrins, permethrin, phenothrin, flumethrin, cyfluthrin, d.d-T80 prarethrin, phthalthrin, transfluthrine, resmethrin, etofenprox, cyphenothrin, pyrethrum extract, fluvalinate, bromopropylate, tetradifon, piperonylbutoxide, synepirin 222, synepirin 500, pyriproxyfene, lufenuron, methoprene, etoxazole, deet, diazinon, fenitrothion, dichlorvos, prothiofos, trichlofon, haloxon, coumaphos, malathion, dimpylate, naled, tetrachlorvinphos, bromofenofos, cythioate, metoxadiazon, carbaryl, fenobucarb, propoxur, diflubenzuron, teflubenzuron, cyromazine, triflumuron, star anise oil, amitraz, triclabendazole, flubendazole, fenbendazole, parbendazole, tiabendazole, hygtomycin B, destomycin A, clorsulon, nitroxynil, diamfenethide, antimony sodium gluconate, levamisole hydrochloride, bithionol, dichlorofen, phenothiazine, piperazine carbon bisulfide, piperazine phosphate, piperazine adipate, piperazine citrate, melarsomine dihydrochloride, melrsonyl, dithiazanine iodide, stibophen, metyridine, disophenol, dietylcarbamazine, diminazene, acrinamine, metronidazole, santonin, pyrantel pamoate, pyrantel, morantel, praziquantel, febantel, bithionol, bunamidine, arecoline, emodepside, metaflumizone and spinosad.

Examples of ectoparasite control agents for animals admixable into at least one of 16-keto aspergillimide, its enantiomers, their mixtures, or their acid addition salts include pyrethroid compounds, neonicotinoid compounds, avermectin compounds, macrolide compounds, phenylpyrazole compounds, phenylpyrrole compounds, organophosphorus

compounds, carbamate compounds, nereistoxin derivatives, organochlorine compounds, benzoyl urea compounds, juvenile hormone analogies, molting hormone analogies, spinosyn compounds, cyclodepsipeptide compounds, sodium channel blockers for nerve cells, insecticidal macrocyclic lactone, γ-aminobutyric acid (GABA) antagonists and ryanodine receptor agonistic compounds. Specific examples of more preferred ectoparasite control agents include ivermectin, selamectin, moxidectin, doramectin, eprinomectin, milbemycin oxime, maduramycin, imidacloprid, dinotefuran, nitenpyram, acetamiprid, thiacloprid, thiamethoxam, clothianidin, sulfoxaflor, fipronil, ethiprole, pyriprole, allethrin, d.d-T allethrin, dl.d-T80 allethrin, pyrethrins, permethrin, phenothrin, flumethrin, cyfluthrin, d.d-T80 prarethrin, phthalthrin, transfluthrine, resmethrin, etofenprox, cyphenothrin, pyrethrum extract, piperonylbutoxide, synepirin 222, synepirin 500, pyriproxyfene, lufenuron, methoprene, etoxazole, deet, diazinon, fenitrothion, dichlorvos, prothiofos, trichlofon, coumaphos, malathion, dimpylate, naled, tetrachlorvinphos, cythioate, metoxadiazon, carbaryl, fenobucarb, propoxur, diflubenzuron, teflubenzuron, cyromazine, triflumuron, star anise oil, amitraz, metaflumizone and spinosad. More preferred examples thereof include ivermectin, imidacloprid, nitenpyram, acetamiprid, thiamethoxam, clothianidin, dinotefuran and sulfoxaflor.

According to another aspect of the present invention, there is provided a composition for use in controlling harmful organisms that comprises, in addition to the above ingredients, a suitable agriculturally and zootechnically acceptable carrier. The composition may be formulated into any suitable dosage forms, for example, emulsifiable concentrates, liquid formulations, suspensions, wettable powders, water dispersible granules, flowables, dusts, DL dusts, granules, micro granule fines, tablets, oils, aerosols, smoking agents, or microcapsules. These dosage forms may be produced as described, for example, in "Noyaku Seizai Gaido (Guide for Pesticide Formulation)" edited by "pesticide Science Society of Japan/Seyoho Kenkyukai (Special Committee on Agricultural Formulation and Application)", Japan Plant Protection Association, 1997.

Carriers usable herein include solid carriers, liquid carriers, gaseous carriers, surfactants, dispersants and other adjuvants for formulations.

Solid carriers include, for example, talc, bentonite, clay, kaolin, diatomaceous earth, vermiculite, white carbon and calcium carbonate.

Liquid carriers include, for example, alcohols such as methanol, n-hexanol and ethylene glycol; ketones such as acetone, methyl ethyl ketone and cyclohexanone; aliphatic hydrocarbons such as n-hexane, kerosine and kerosene; aromatic hydrocarbons such as toluene, xylene and methyl naphthalene; ethers such as diethyl ether, dioxane and tetrahydrofuran; esters such as ethyl acetate; nitriles such as acetonitrile and isobutyronitrile; acid amides such as dimethylformamide and dimethylacetamide; vegetable oils such as soybean oil and cotton seed oil; dimethylsulfoxide; and water.

Gaseous carriers include, for example, LPG, air, nitrogen, carbon dioxide and dimethyl ether.

Surfactants or dispersants usable for emulsifying, dispersing, or spreading include, for example, alkylsulfuric esters, alkyl(aryl)sulfonic acid salts, polyoxyalkylene alkyl(aryl) ethers, polyhydric alcohol esters and lignin sulfonic acid salts.

Adjuvants usable for improving the properties of formulations include, for example, carboxymethylcellulose, gum arabic, polyethylene glycol and calcium stearate.

The above carriers, surfactants, dispersants and adjuvants may be used either solely or in a combination according to need.

The content of the active ingredients in the composition according to the present invention is not particularly limited but is 0.1 to 99.9% by weight, preferably 0.2 to 80% by weight, in terms of total content. The mixing ratio between the 16-keto aspergillimide and the other harmful organism control agent(s) may vary in a wide range. In general, the composition according to the present invention contains 0.1 to 80% by weight of the 16-keto aspergillimide.

Compositons for use in controlling harmful organisms, which further comprise agriculturally and zootechnically acceptable carriers, in a preferred embodiment include:

(1) a composition in a wettable powder form comprising 0.1 to 80% by weight of the 16-keto aspergillimide, 0.1 to 80% by weight of an insecticide as the other harmful organism control agent, 0.6 to 30% by weight of a wetting agent and a dispersant, and 20 to 95% by weight of an extender,

(2) a composition in a water dispersible granule form comprising 0.1 to 80% by weight of the 16-keto aspergillimide, 0.1 to 80% by weight of an insecticide as the other harmful organism control agent, 0.6 to 30% by weight of a wetting agent, a dispersant, and a binder, and 20 to 95% by weight of an extender,

(3) a composition in a flowable form comprising 0.1 to 80% by weight of the 16-keto aspergillimide, 0.1 to 80% by weight of an insecticide as the other harmful organism control agent, 5 to 40% by weight of a dispersant, a thickening agent, an antifreezing agent, an antiseptic, and an antifoaming agent, and 20 to 94% by weight of water,

(4) a composition in an emulsifiable concentrate form comprising 0.1 to 80% by weight of the 16-keto aspergillimide, 0.1 to 80% by weight of an insecticide as the other harmful organism control agent, 1 to 30% by weight of an emulsifier and an emulsion stabilizer, and 20 to 97% by weight of an organic solvent,

(5) a composition in a dust form comprising 0.1 to 80% by weight of the 16-keto aspergillimide, 0.1 to 80% by weight of an insecticide as the other harmful organism control agent, and 70 to 99.8% by weight of an extender,
(6) a composition in a DL dust (low drift dust) form comprising 0.1 to 80% by weight of the 16-keto aspergillimide, 0.1 to 80% by weight of an insecticide as the other harmful organism control agent, and 70 to 99.8% by weight of an extender,
(7) a composition in a micro granule fine form comprising 0.1 to 80% by weight of the 16-keto aspergillimide, 0.1 to 80% by weight of an insecticide as the other harmful organism control agent, 0.2 to 10% by weight of a solvent or a binder, and 70 to 99.6% by weight of an extender,
(8) a composition in a granule form comprising 0.1 to 80% by weight of the 16-keto aspergillimide, 0.1 to 80% by weight of an insecticide as the other harmful organism control agent, 0.5 to 30% by weight of a granulation assistant (a surfactant) and a binder, and 20 to 98% by weight of an extender, and
(9) a composition in a microcapsule form comprising 0.1 to 80% by weight of the 16-keto aspergillimide, 0.1 to 80% by weight of an insecticide as the other harmful organism control agent, 1 to 50% by weight of a coating agent, an emulsifier, a dispersant, and an antiseptic, and 20 to 98% by weight of water.
Preferably, the compositon for use in controlling harmuful organisms further comprising an agricultrually and zootechnically acceptable carrier is the composition (8).

The composition for use in controlling harmful organisms adapted for use in animals may be formulated into a variety of dosage forms acceptable as ectoparasite control agents, for example, liquid formulations, sprays, foam formulations, tablets, granules, fine subtilaes, dust, capsules, tablets, chewable formulations, injections, suppositories, creams, shampoos, rinses, resin agents, fumigants and poison baits. Among them, liquid formulations are particularly preferred.

Carriers usable in the preparation of the composition for use in controlling harmful organisms adapted for use in animals according to the present invention include liquid carriers, solid carriers, gaseous carriers, surfactants, dispersants and other adjuvants for formulations.

Liquid carriers include, for example, alcohols such as methanol, ethanol, isopropanol, polyethylene glycol, propylene glycol, dipropylene glycol, tripropylene glycol and glycerin; ketones such as acetone and methyl ethyl ketone; aromatic hydrocarbons such as benzyl alcohol, benzene, toluene, xylene, ethylbenzene, and methyl naphthalene, aliphatic hydrocarbons such as paraffin, n-hexane, cyclohexane, kerosine and kerosine, ethers such as diethylene glycol monoethyl ether, diethylene glycol monomethyl ether, diisopropyl ether, diethyl ether, dioxane, and tetrahydrofuran, esters such as propylene carbonate, ethyl acetate, butyl acetate, benzyl benzoate, isopropyl myristate, and fatty acid ester of propylene glycol, nitriles such as acetonitrile and isobutyronitrile, amides such as dimethylformamide, dimethylacetamide, and N-methylpyrrolidone, halogenated hydrocarbons such as dichloromethane, trichloroethane, and carbon tetrachloride, animal and vegetable oils such as soybean oils and cotton seed oils, dimethylsulfoxide, silicone oils, higher fatty acids, glycerol formal and water.

Solid carriers include, for example, impalpable powders and particles of clays such as kaolin clay, diatomaceous earth, bentonite, and acid white clay, synthetic hydrous silicon oxide, talc, ceramic, other inorganic minerals such as selenite, quartz, sulfur, activated carbon, calcium carbonate, and hydrated silica, starch, lactose, and synthetic polymers such as vinyl chloride polymer and polyurethane.

Conventional emulsifiers, dispersants, spreaders, wetting agents, suspending agents, preserving agents, propellants and the like may be further incorporated in the liquid formulation. Further, conventional coating film forming agents may be incorporated. Surfactants for emulsification, dispersion, spreading or the like include, for example, soaps, polyoxyalkylene alkyl (aryl) ethers, polyoxyethylene alkyl allyl ethers, polyoxyethylene fatty acid esters, higher alcohols and alkyarylsulfonates. Dispersants include, for example, casein, gelatin, polysaccharides, lignin derivatives, saccharides and synthetic water soluble polymers. Spreaders/wetting agents include, for example, glycerin and polyethylene glycol. Susspending agents include, for example, casein, gelatin, hydroxypropylcellulose and gum arabic. Stabilizers include, for example, phenolic antioxidants such as BHT and BHA, amine antioxidants such as diphenylamine and organic sulfur antioxidants. Preserving agents include, for example, methyl p-oxybenzoate, ethyl p-oxybenzoate, propyl p-oxybenzoate and butyl p-oxybenzoate. The carriers, surfactants, dispersants and adjuvants may if necessary be used solely or in a combination of two or more. Perfumes, synergists and the like may also be incorporated. The content of the active ingredients in the composition for use in controlling harmful organisms adapted for use in animals is generally 1 to 75% by weight for the liquid formulation.

Carriers usable for the preparation of creams include, for example, nonvolatiel hydrocarbons such as liquid paraffin, lanolin hydrogenated fats and oils, higher fatty acids, fatty acid esters, animal and vegetable oils, silicone oils and water. The emulsifiers, humectant, antioxidants, perfumes, borax and ultraviolet absorbers may if necessary be used solely or in a combination of two or more. Emulsifiers include, for example, fatty acid sorbitan, polyoxyethylene alkyl ethers and fatty acid polyoxyethylene. The content of the active ingredients in the composition for use in controlling harmful organisms adapted for use in animals is generally 0.5 to 70% by weight for the cream.

The capsules, pills or tablets may be used in such a manner that the active ingredients in the composition according to the present invention is divided into suitable small portions, the small portion is mixed with a diluting solution or a carrier such as starch, lactose, or talc, a disintegrator such as magnesium stearate and/or a binder is further added thereto, and, if necessary, the mixture is tabletted.

Carriers for the preparation of injections should be prepared as an aseptic solution. The aseptic solution may contain other substances, for example, a salt or glucose in an amount enough to be isotonicated with blood. Carriers usable herein include organic solvents, for example, esters such as glycerides, benzyl benzoate, isopropyl myristate, and fatty acid derivatives of propylene glycol, N-methylpyrrolidone, and glycerol formal. The content of the active ingredients in the composition for use in controlling harmful organisms adapted for use in animals is generally 0.01 to 10% by weight for the injection.

Carriers usable for the preparation of resin agents include, for example, vinyl chloride polymers and polyurethane. Plasticizers such as phthalic acid esters, adipic acid esters, and stearic acid may be added to these bases. After kneading of the active ingredients according to the present invention into the base, the kneaded product is molded, for example, by injection molding, extrusion, or press molding. The molded product may also be further properly subjected to molding, cutting or the like to obtain an ear tag for animals or insecticidal collar for animals.

Carriers usable for toxic baits include bait substances and attractants, for example, farina such as wheat flour and corn flour, starch such as corn starch and potato starch, saccharides such as granulated sugar, malt sugar, and honey, food flavors such as glycerin, onion flavor, and milk flavor, animal powders such as pupal powder and fish powder, and various pheromones. The content of the active ingredients in the composition for use in controlling harmful organisms adapted for use in animals is generally 0.0001 to 90% by weight for the toxic bait.

In the composition for use in controlling harmful organisms according to the present invention, a method may be adopted in which a first composition containing, as active ingredient, only a first active ingredient of the composition according to the present invention, i.e., at least one of 16-keto aspergillimide, its enantiomers, their mixture, or their acid addition salts, and a second composition containing, as active ingredient, only a second active ingredient of the composition according to the present invention, i.e., at least one of other harmful organism control agent, are prepared and, in use, these two compositions are mixed together on site.

According to another preffered aspect of the present invention, there is provided a combination comprising as active ingredients at least one of 16-keto aspergillimide, its enantiomers, their mixture, or their acid addition salts and at least one of other harmful organism control agents.

In another preferred embodiment of the present invention, in the combination, at least one of 16-keto aspergillimide, its enantiomers, their mixture, or their acid addition salts is provided as a first composition containing the at least one substance as an active ingredient, and at least one of other harmful organism control agents is provided as a second composition containing the at least one of other harmful organism control agents as an active ingredient. In this case, as with the above composition for use in controlling harmful organisms, the first and second compositions may be in any desired dosage form using a suitable carrier or adjuvant. The combination may also be provided in a form like a drug set.

According to still another aspect of the present invention, there is provided a method for protecting useful plants or animals from harmful organisms, the method comprising applying at least one of 16-keto aspergillimide, its enantiomers, their mixture, or their acid addition salts and at least one of other harmful organism control agents simultaneously or separately from each other (preferably simultaneously) to an area to be treated.

In this method, applying "simultaneously" embraces a case where at least one of 16-keto aspergillimide, its enantiomers, their mixture, or their acid addition salts and at least one of other harmful organism control agents are mixed together before application to an area to be treated and the mixture is applied to the object area. On the other hand, applying "separately" embraces a case where, without previously mixing at least one of 16-keto aspergillimide, its enantiomers, their mixture or their acid addition salts and at least one of other harmful organism control agents together, at least one of 16-keto aspergillimide, its enantiomers, their mixure, or their acid addition salts is applied before the application of at least one of other harmful organism control agents, and a case where, without previously mixing at least one of 16-keto aspergillimide, its enantiomers, their mixture, or their acid addition salts and at least one of other harmful organism control agents together, at least one of 16-keto aspergillimide, its enantiomers, their mixture, or their acid addition salts is applied after the application of at least one of other harmful organism control agents.

According to another aspect of the present invention, there is provided a method for protecting useful plants from harmful organisms, comprising applying

(1) a first composition comprising at least one of 16-keto aspergillimide, its enantiomers, their mixture, or their acid addition salts as an active ingredient and
(2) a second composition comprising at least one of other harmful organism control agents as active ingredient to an area to be treated.

According to still another aspect of the present invention, there is provided a method for protecting useful plants or animals from harmful organisms, comprising treating an object harmful organism, an object useful plant, or a seed, a soil, or a cultivation carrier of the object useful plant, or an object animal, preferably an object useful plant, a soil of the object useful plant, or an object animal with an effective amount of the composition or the combination according to the present invention as such or after dilution.

According to a further aspect of the present invention, there is provided use of the harmful organisms control composition or the combination according to the present invention for the protection of useful plants or animal from harmful organisms.

Preferred methods for treating the object harmful organism, the object useful plant, or the seed, soil, or cultivation materials of the object useful plant with the harmful organism control composition according to the present invention include spreading treatment, water surface treatment, soil treatment (such as mixing or drenching), nursery box treatment, surface treatment (coating, dust coating, or covering), or fumigation treatment (treatment in a closed space such that soil is covered with a polymer film after injection into soil). More preferred are water surface treatment, soil treatment, nursery box treatment, or surface treatment.

In the application to plants by spreading, the amount of the composition used for the application is 0.1 to 10 kg, preferably 1 g to 1 kg in terms of the amount of the active ingredient in the composition of the present invention based on 10 ares of cultivated land.

Methods for treating seeds, roots, tubers, bulbs, and rhizomes of plants include, for example, dipping, dust coating, smearing, spraying, pelleting, coating and fumigation methods.

The dipping is a method in which seeds are dipped in a chemical solution. The dust coating is classified into two types, i.e., a dry dust coating method in which a powdery chemical is adhered onto dry seeds, and a wet dust coating method in which a powdery chemical is adhered onto seeds which have been lightly soaked in water. The smearing is a method in which a suspended chemical is coated on the surface of seeds within a mixer. The spraying is a method in which a suspended chemical is sprayed onto the surface of seeds. The pelleting is a method in which a chemical is mixed with a filler when seeds, together with a filler, are pelleted to form pellets having given size and shape. The coating is a method in which a chemical-containing film is coated onto seeds. The fumigation is a method in which seeds are sterilized with a chemical which has been gasified within a hermetically sealed vessel.

Preferred treating methods using the composition according to the present invention include dipping, dust coating, smearing, spraying, pelleting and coating methods.

The composition can also be applied to, in addition to seeds, germinated plants which are transplanted after germination or after budding from soil, and embryo plants. These plants can be protected by the application of the whole or a part thereof by dipping before transplantation.

The amount of the composition applied to seeds of plants is not particularly limited but is preferably 1 g to 10 kg, more preferably 100 g to 1 kg in terms of the amount of the active ingredient in the composition according to the present invention based on 100 kg of seeds.

The method for applying the composition according to the present invention to soil is not particularly limited but is preferably as follows.

An example of such methods is one in which granules containing the composition according to the present invention are applied into soil or on soil. Particularly preferred soil application methods include spreading, stripe application, groove application and planting hole application.

Drenching of soil with a solution prepared by emulsifying or dissolving the composition according to the present invention in water is also an advantageous soil application method.

Examples of other preferred soil application methods include application into a nutrient solution in nutrient solution culture systems such as water culture and solid medium culture, for example, sand culture, NFT (nutrient film technique), or rock wool culture, for the production of vegetables and flowering plants, and application into a nursery box (bed soil mixing) for rice rasiing seedling. A method may also be adopted in which the composition according to the present invention can be applied directly to artificial culture soil containing vermiculite and a solid medium containing an artificial mat for nursery.

The amount of the composition according to the present invention applied to the water surface, nursery box, or soil is not particularly limited but is preferably 0.1 g to 10 kg, preferably 1 g to 1 kg, in terms of the amount of the active ingredient based on 10 ares of cultivated land.

Preferred methods for applying the composition or combination according to the present invention to an object animal include oral or injection administration into the body of an object animal, administration onto the whole or a part of the body surface of an object animal, covering of a place where invasion, parasitism, and migration of ectoparasites, and application to animal-rearing houses are expected,.

The composition or combination according to the present invention as such may be used, or alternatively may be diluted with water, a liquid carrier, a commercially available shampoo, a rinse, a feed, an underlayment for animal-rearing houses or the like.

[EXAMPLES]

The present invention is further illustrated by the following Examples that are not intended as a limitation of the invention.

Preparation Examples

Preparation Example 1 [Wettable powder]

| | |
|---|---|
| 16-Keto aspergillimide | 10 wt% |
| Imidacloprid | 20 wt% |
| Clay | 50 wt% |
| White carbon | 2 wt% |
| Diatomaceous earth | 13 wt% |
| Calcium lignin sulfonate | 4 wt% |
| Sodium lauryl sulfate | 1 wt% |

The above ingredients were intimately mixed together, and the mixture was ground to prepare wettable powder.

Preparation Example 2 [Water dispersible granule]

| | |
|---|---|
| 16-Keto aspergillimide | 10 wt% |
| Imidacloprid | 20 wt% |
| Clay | 60 wt% |
| Dextri n | 5 wt% |
| Alkylmaleic acid copolymer | 4 wt% |
| Sodium lauryl sulfate | 1 wt% |

The above ingredients were homogeneously ground and intimately mixed together. Water was added to the mixture, followed by thorough kneading. Thereafter, the kneaded product was granulated and dried to prepare a water dispersible granule.

Preparation Example 3 [Floables]

| | |
|---|---|
| 16-Keto aspergillimide | 5 wt% |
| Imidacloprid | 20 wt% |
| POE polystyrylphenyl ether sulfate | 5 wt% |
| Propylene glycol | 6 wt% |
| Bentonite | 1 wt% |
| 1% aqueous xanthan gum solution | 3 wt% |
| PRONALEX-300<br>(Toho Chemical Industry Co., Ltd.) | 0.05 wt% |
| ADDAC 827<br>(K.I. Chemical Industry Co., Ltd.) | 0.02 wt% |
| Water | To 100 wt% |

All the above ingredients except for the 1% aqueous xanthan gum solution and a suitable amount of water were premixed together, and the mixture was then ground by a wet grinding mill. Thereafter, the 1% aqueous xanthan gum solution

and the remaining water were added to the ground product to prepare 100 wt% floables.

Preparation Example 4 [Emulsifiable concentrate]

| | |
|---|---|
| 16-Keto aspergillimide | 2 wt% |
| Imidacloprid | 13 wt% |
| N,N-dimethylformamide | 20 wt% |
| Solvesso 150 (Exxon Mobil Corporation) | 55 wt% |
| Polyoxyethylene alkyl aryl ether | 10 wt% |

The above ingredients were intimately mixed together and dissolved to prepare an emulsifiable concentrate.

Preparation Example 5 [Dust]

| | |
|---|---|
| 16-Keto aspergillimide | 0.5 wt% |
| Imidacloprid | 1.5 wt% |
| Clay | 60 wt% |
| Talc | 37 wt% |
| Calcium stearate | 1 wt% |

The above ingredients were intimately mixed together to prepare dust.

Preparation Example 6 [DL dust]

| | |
|---|---|
| 16-Keto aspergillimide | 1 wt% |
| Imidacloprid | 1 wt% |
| DL clay | 95.5 wt% |
| White carbon | 2 wt% |
| Light liquid paraffin | 0.5 wt% |

The above ingredients were intimately mixed together to prepare DL dust.

Preparation Example 7 [Micro granules fine

| | |
|---|---|
| 16-Keto aspergillimide | 1 wt% |
| Imidacloprid | 1 wt% |
| Carrier | 94 wt% |
| White carbon | 2 wt% |
| Hisol SAS-296 | 2 wt% |

The above ingredients were intimately mixed together to prepare micro granules.

Preparation Example 8 [Granules]

| | |
|---|---|
| 16-Keto aspergillimide | 2 wt% |
| Imidacloprid | 3 wt% |
| Bentonite | 40 wt% |
| Talc | 10 wt% |
| Clay | 43 wt% |
| Calcium lignin sulfonate | 2 wt% |

The above ingredients were homogeneously ground and intimately mixed together. Water was added to the mixture, followed by thorough kneading. Thereafter, the kneaded product was granulated and dried to prepare granules.

Preparation Example 9 [Microcapsules]

| | |
|---|---|
| 16-Keto aspergillimide | 2 wt% |
| Imidacloprid | 3 wt% |
| Urethane resin | 25 wt% |
| Emulsifying dispersant | 5 wt% |
| Antiseptic | 0.2 wt% |
| Water | 64.8 wt% |

The above ingredients were polymerized by interfacial polymerization to form a urethane resin film on the surface of 16-Keto aspergillimide particles and imidacloprid particles and thus prepare microcapsules.

Preparation Example 10 [Granules]

| | |
|---|---|
| 16-Keto aspergillimide | 3 wt% |
| Probenazole | 24 wt% |
| Sodium lauryl sulfate | 1 wt% |
| Bentonite | 2 wt% |
| Calcium stearate | 1 wt% |
| PVA | 2 wt% |
| Clay | 67 wt% |

The above ingredients were homogeneously ground and intimately mixed together. Water was added to the mixture, followed by thorough kneading. Thereafter, the kneaded product was granulated and dried to prepare granules.

Preparation Example 11 [Liquid drops]

| | |
|---|---|
| 16-Keto aspergillimide | 3.3 wt% |
| Imidacloprid | 16.7 wt% |
| Diethylene glycol monoethyl ether | 66.7 wt% |
| Ethanol | 13.3 wt% |

The above ingredients were intimately mixed together to prepare liquid drops.

Test Examples

<Soil drenching application test>

Test Example 1: Laodelphax striatellus soil drenching application test

Test solutions adjusted to a predetermined concentration (10% aqueous acetone solution) were applied to rice seedling in a pot by soil drenching. The rice seedling was allowed to stand for 3 days. Second instar larvae were released and was then allowed to stand in a humidistat chamber (light period 16 hr - dark period 8 hr) (25°C). Three days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

Further, theoretical values, which do not exhibit a synergistic effect, were calculated by the following Colby's formula, and the results are shown in Table 2.

$$\text{Colby's formula: Theoretical value (\%)} = 100 - (A \times B)/100$$

wherein A: 100 - (death rate when application was performed only with 16-keto aspergillimide); and
B: 100 - (death rate when application was performed only with each of imidacloprid, dinotefuran, or fipronil)

Method for determining synergistic effect

When the insecticidal effect against Laodelphax striatellus for the admixtures (Table 1 below) exceeded the theoretical value calculated by the Colby's formula shown in Table 2, the admixture was determined to have a synergistic effect.
All the tested admixtures had death rate beyond the theoretical values, demonstrating that they had a synergistic effect.
[Table 1]

Table 1: Death rate (%) of single agent and admixture against Laodelphax striatellus

| 16-Keto aspergillimide / Other insecticide | 0 mg/seedling | 0.2 mg/seedling |
|---|---|---|
| - | 0 | 30 |
| Imidacloprid 0.005 mg/seedling | 5 | 60 |
| Dinotefuran 0.002 mg/seedling | 4 | 40 |
| Fipronil 0.002 mg/seedling | 5 | 46 |

[Table 2]

Table 2: Theoretical value (%) calculated by the Colby's formula

| 16-Keto aspergillimide / Other insecticide | 0 mg/seedling | 0.2 mg/seedling |
|---|---|---|
| - | 0 | 30 |
| Imidacloprid 0.005 mg/seedling | 5 | 34 |
| Dinotefuran 0.002 mg/seedling | 4 | 33 |
| Fipronil 0.002 mg/seedling | 5 | 34 |

<Foliage application test>

Test Example 2: Foliage application test for Laodelphax striatellus

Test solutions adjusted to a predetermined concentration (10% aqueous acetone solution) were used for foliage application of the rice seedlings in a pot. The rice seedlings were then air dried, and second instar larvae were released and was then allowed to stand in a humidistat chamber (light period 16 hr - dark period 8 hr) (25°C). Three days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

Further, theoretical values, which do not exhibit a synergistic effect, were calculated by the following Colby's formula,

and the results are shown in Table 4.

$$\text{Colby's formula: Theoretical value (\%)} = 100 - (A \times B)/100$$

wherein A: 100 - (death rate when application was performed only with 16-keto aspergillimide); and
B: 100 - (death rate when application was performed only with each of imidacloprid, clothianidin, or fipronil)

Method for determining synergistic effect

When the insecticidal effect against Laodelphax striatellus for the admixtures (Table 3 below) exceeded the theoretical value calculated by the Colby's formula shown in Table 4, the admixture was determined to have a synergistic effect.
All the tested admixtures had death rate beyond the theoretical values, demonstrating that they had a synergistic effect.
[Table 3]

Table 3: Death rate (%) of single agent and admixture against Laodelphax striatellus

| 16-Keto aspergillimide / Other insecticide | 0 ppm | 2.5 ppm |
|---|---|---|
| - | 0 | 65 |
| Imidacloprid 0.313 ppm | 5 | 86 |
| Fipronil 0.313 ppm | 21 | 82 |
| Clothianidin 0.313 ppm | 4 | 83 |

[Table4]

Table 4: Theoretical value (%) calculated by the Colby's formula

| 16-Keto aspergillimide / Other insecticide | 0 ppm | 2.5 ppm |
|---|---|---|
| - | 0 | 65 |
| Imidacloprid 0.313 ppm | 5 | 67 |
| Fipronil 0.313 ppm | 21 | 72 |
| Clothianidin 0.313 ppm | 4 | 66 |

Test Example 3: Foliage application test for Aphids gossypii Test solutions adjusted to a predetermined concentration (50% aqueous acetone solution; 0.05% Tween 20 added) were applied to a leaf disk having a diameter of 2.0 cm cut out from a cucumber grown in a pot. The leaf disk was then air dried, and first instar larvae were released and was then allowed to stand in a humidistat chamber (light period 16 hr - dark period 8 hr) (25°C). Three days after the release, the larvae were observed for survival or death, and the death rate of larvae was calculated by the following equation, and the results are shown in Table 5 below.

$$\text{Death rate (\%)} = \{\text{number of dead larvae}/(\text{number of survived larvae} + \text{number of dead larvae})\} \times 100$$

Further, theoretical values, which do not exhibit a synergistic effect, were calculated by the following Colby's formula, and the results are shown in Table 6.

$$\text{Colby's formula: Theoretical value (\%)} = 100 - (A \times B)/100$$

wherein A: 100 - (death rate when application was performed only with 16-keto aspergillimide); and
B: 100 - (death rate when application was performed only with a compound represented by formula (I) described in Japanese Patent No. 4015182, wherein $Het_1$ represents 3-pyridyl; $R_1$ represents hydroxyl; both $R_2$ and $R_3$ represent cyclopropylcarbonyloxy; and $R_4$ represents hydroxyl.

Method for determining synergistic effect

When the insecticidal effect against Aphis gossypii for the admixtures (Table 5 below) exceeded the theoretical value calculated by the Colby's formula shown in Table 6, the admixture was determined to have a synergistic effect. All the tested admixtures had death rate beyond the theoretical values, demonstrating that they had a synergistic effect.
[Table 5]

Table 5: Death rate (%) of single agent and admixture against Aphis gossypii

| 16-Keto aspergillimide / Other insecticide | 0 ppm | 1.25 ppm |
|---|---|---|
| - | 0 | 26 |
| Compound of formula (I) 0.01ppm | 40 | 78 |

[Table6]

Table 6: Theoretical value calculated by the Colby's formula (%)

| 16-Keto aspergillimide / Other insecticide | 0 ppm | 1.25ppm |
|---|---|---|
| - | 0 | 26 |
| Compound of formula (I) 0.01 ppm | 40 | 56 |

Test Example 4: Foliage application test for Nilaparvata lugens (imidacloprid low-sensitive strain)

Test solutions adjusted to a predetermined concentration (50% aqueous acetone solution) applied to rice seedlings in a pot for foliage application. After the application, second instar larvae were released, and the rice seedling was then allowed to stand in a humidistat chamber (light period 16 hr - dark period 8 hr) (25°C). Six days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

For comparison, a test was carried out in the same manner as described above, except that a Nilaparvata lugens strain which is highly sensitive to imidacloprid was used. The results are shown in Table 8. As shown in Table 7, for 16-keto aspergillimide, the death rate for the Nilaparvata lugens strain which is low-sensitive to imidacloprid was the same as that for the Nilaparvata lugens strain which is high-sensitive to imidacloprid shown in Table 8 below, and, thus, it is expected to attain a high insecticidal effect against imidacloprid-sensitive Nilaparvata lugens by admixing 16-keto aspergillimide with imidacloprid.

Regarding the origin of the test insect pests, Nilaparvata lugens which is low-sensitive to imidacloprid was an insect

which had been collected outdoors in Kumamoto Prefecture, 2007, and Nilaparvata lugens which is sensitive to imidacloprid was an insect which had been colleced in Kagoshima Prefecture and was then subjected to indoor successive raising.

[Table 7]

Table 7: Insecticidal effect of 16-keto aspergillimide and imidacloprid against Nilaparvata lugens which is low-sensitive to imidacloprid

| Preparation name | Treatment concentration (ppm) | Death rate (%) |
|---|---|---|
| 16-Keto aspergillimide | 50 | 100 |
| | 20 | 95 |
| | 5 | 60 |
| Imidacloprid | 20 | 85 |
| | 5 | 55 |
| | 1.25 | 15 |

[Table 8]

Table 8: Insecticidal effect of 16-keto aspergillimide and imidacloprid against Nilaparvata lugens which is sensitive to imidacloprid

| Preparation name | Treatment concentration (ppm) | Death rate (%) |
|---|---|---|
| 16-Keto aspergillimide | 50 | 100 |
| | 20 | 100 |
| | 5 | 95 |
| Imidacloprid | 20 | 100 |
| | 5 | 100 |
| | 1.25 | 100 |

Test Example 5: Tickcidal effect against Haemaphysalis longicornis

Acetone solutions (30 μl) adjusted to predetermined concentrations were placed in 4 mL-volume glass vials. The vials were placed on a shaker, and a dry film of the compound was prepared on the inner wall of the vials. The vials were dried for 24 hr or more. Larvae (10 or more heads) were released in the vials, and the vials were lidded. The vials were then left to stand in a humidistat chamber (25°C, humidity 85%, fully darkened conditions). Two days after the release, the larvae were observed for survival or death, and the death rate of larvae was calculated by the following equation. The results are shown in Tables 9 and 10 below. The test was duplicated.

$$\text{Death rate (\%)} = \{\text{number of dead larvae}/(\text{number of survived larvae} + \text{number of dead larvae})\} \times 100$$

Further, theoretical values, which do not exhibit a synergistic effect, were calculated by the following Colby's formula, and the results are shown in Tables 11 and 12 below.

$$\text{Colby's formula: Theoretical value (\%)} = 100 - (A \times B)/100$$

wherein A: 100 - (death rate when application was performed only with 16-keto aspergillimide); and
B: 100 - (death rate when application was performed only with each of imidacloprid, chlothianidin, dinotefuran, acetamiprid, thiamethoxam, sulfoxaflor, or ivermectin.

Method for determining synergistic effect

When the tickcidal effect against Haemaphysalis longicornis for the admixtures (Tables 9 and 10) exceeded the theoretical value calculated by the Colby's formula shown in Tables 11 and 12, the admixture was determined to have a synergistic effect.
All the tested admixtures had death rates beyond the theoretical values, demonstrating that they had a synergistic effect.
[Table 9]

Table 9: Death rate (%) of single agent and admixture against Haemaphysalis longicornis

| 16-Keto aspergillimide / Other insecticide, ectoparasiticide | 0 ppm | 1 ppm | 2 ppm |
|---|---|---|---|
| - | 0 | 10 | 17 |
| Dinotefuran | | | |
| 30 ppm | 12 | 24 | 28 |
| 50 ppm | 14 | 33 | 41 |
| Nitenpyram | | | |
| 40 ppm | 12 | 46 | 44 |
| Imidacloprid | | | |
| 1.5 ppm | 26 | 63 | 68 |
| 3 ppm | 33 | 71 | 89 |
| Acetamiprid | | | |
| 1.5 ppm | 24 | 32 | 66 |
| 3 ppm | 55 | 60 | 67 |

[Table 10]

Table 10: Death rate (%) of single agent and admixture against Haemaphysalis longicornis

| 16-Keto aspergillimide / Other insecticide, ectoparasiticide | 0 ppm | 1 ppm | 2 ppm |
|---|---|---|---|
| - | 0 | 14 | 20 |
| Chlothianidin | | | |
| 5 ppm | 35 | 48 | - |
| 10 ppm | 62 | 74 | 94 |
| Sulfoxaflor | | | |
| 75 ppm | 48 | - | 100 |
| 150 ppm | 60 | - | 100 |
| Thiamethoxam | | | |
| 30 ppm | 21 | 56 | 70 |
| 60 ppm | 26 | 81 | 79 |
| Ivermectin | | | |
| 200 ppm | 0 | 22 | 39 |
| 400 ppm | 0 | 19 | 26 |

[Table 11]

Table 11: Theoretical value (%) by Colby's formula

| 16-Keto aspergillimide / Other insecticide, ectoparasiticide | 0 ppm | 1 ppm | 2 ppm |
|---|---|---|---|
| - | 0 | 10 | 17 |
| Dinotefuran | | | |
| 30 ppm | 12 | 21 | 27 |
| 50 ppm | 14 | 22 | 28 |
| Nitenpyram | | | |
| 40 ppm | 12 | 21 | 27 |
| Imidacloprid | | | |
| 1.5 ppm | 26 | 33 | 39 |
| 3 ppm | 33 | 40 | 45 |
| Acetamiprid | | | |
| 1.5 ppm | 24 | 32 | 37 |
| 3 ppm | 55 | 59 | 62 |

[Table 12]

Table 12: Theoretical value (%) by Colby's formula

| 16-Keto aspergillimide / Other insecticide, ectoparasiticide | 0 ppm | 1 ppm | 2 ppm |
|---|---|---|---|
| - | 0 | 14 | 20 |
| Chlothianidin | | | |
| 5 ppm | 35 | 44 | - |
| 10 ppm | 62 | 68 | 70 |
| Sulfoxaflor | | | |
| 75 ppm | 48 | - | 59 |
| 150 ppm | 60 | - | 68 |
| Thiamethoxam | | | |
| 30 ppm | 21 | 32 | 37 |
| 60 ppm | 26 | 36 | 40 |
| Ivermectin | | | |
| 200 ppm | 0 | 14 | 20 |
| 400 ppm | 0 | 14 | 20 |

Test Examples 6: Tickicidal effect against Haemalphysalis longicornis on body surface of mouse

Skin hair on the back of mouse (ICR, male, 5 week olds) was clipped in a diameter of about 2 cm, and a 15-mL polystyrene conical tube cut to have a height of about 1.5 cm was bonded to the body surface of the mouse with an instant adhesive. Test solutions (20 $\mu$l) adjusted with ethanol were dropped on the body surface of mice within the bonded tube. After thorough drying, larvae (10 or more heads) were released within the tube, and the tube was lidded. Three days after the release, the larvae were observed for survival or death, and the death rate of larvae was calculated by the following equation. The results are shown in Table 13. The test was hexaplicated.

$$\text{Death rate (\%)} = \{\text{number of dead larvae}/(\text{number of survived larvae} + \text{number of dead larvae})\} \times 100$$

Further, theoretical values, which do not exhibit a synergistic effect, were calculated by the following Colby's formula, and the results are shown in Table 14.

$$\text{Colby's formula: Theoretical value (\%)} = 100 - (A \times B)/100$$

wherein A: 100 - (death rate when application was performed only with 16-keto aspergillimide); and
B: 100 - (death rate when application was performed only with imidacloprid)

Method for determining synergistic effect

When the tickcidal effect against Haemaphysalis longicornis for the admixtures (Table 13 below) exceeded the theoretical value calculated by the Colby's formula shown in Table 14, the admixture was determined to have a synergistic

effect.
The tested admixtures had death rate beyond the theoretical values, demonstrating that they had a synergistic effect.

[Table 13]

Table 13: Death rate (%) of single agent and admixture against Haemaphysalis longicornis

| 16-Keto aspergillimide / Other insecticide, ectoparasiticide | 0μg/2cm² | 1 μg/2cm² | 2μg/2cm² |
|---|---|---|---|
| - | 0 | 22 | 57 |
| Imidacloprid 6μg/2cm² | 22 | 81 | 86 |

[Table 14]

Table 14: Theoretical value (%) by Colby's formula

| 16-Keto aspergillimide / Other insecticide, ectoparasiticide | 0μg/2cm² | 1μg/2cm² | 2μg/2cm² |
|---|---|---|---|
| - | 0 | 22 | 57 |
| Imidacloprid 6μg/2cm² | 22 | 40 | 67 |

Reference Test Examples

<Root dipping application test for single agent of 16-keto aspergillimide>

Reference Test Example 1: Insecticidal effect against Laodelphax striatellus

A test solution adjusted to a predetermined concentration (10% aqueous acetone solution) was applied to the root of wheat seedlings in water culture. After the absorption of the test solution from the root for 3 days, 10 second instar larvae were released, and the wheat seedlings were then allowed to stand in a humidistat chamber (light period 16 hr - dark period 8 hr) (25°C). Three days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

As is apparent from Table 15, the systemic insecticidal activity of 16-keto aspergillimide was much higher than that of a control compound, Asperparaline A.

The 16-keto aspergillimide and Asperparaline A used in this test were obtained according to methods described in The Journal of Antibiotics, 1997, 50(10), 840-846. This is true of the folloiwng test examples.

[Table 15]

Table 15: Death rate (%) for each amount of test chemical compound

| | Amount of test chemical compound (μg/seedling) |
|---|---|
| Test chemical compound | 0.5 |
| 16-Keto aspergillimide | 100 |
| Asperparaline A | 0 |

Reference Test Example 2: Insecticidal effect against Triqonotvlus caelestialium

A test solution adjusted to a predetermined concentration (10% aqueous acetone solution) was applied to the root of wheat seedlings in water culture. After the absorption of the test solution from the root for 3 days, second instar larvae were released, and the wheat seedlings were then allowed to stand in a humidistat chamber (light period 16 hr - dark period 8 hr) (25°C). Three days after the release, the larvae were observed for survival or death, and the death rate of larvae was calculated in the same manner as in Reference Example 1. The test was duplicated. As is apparent from Table 16 below, 16-keto aspergillimide exhibited systemic insecticidal activity against Trigonotylus caelestialium.

<Soil drenching application test for single agent of 16-keto aspergillimide >

Reference Test Example 3: Insecticidial effect against Nilaparvata lugens

A test solution adjusted to a predetermined concentration (10% aqueous acetone solution) was applied to rice seedlings in a pot by soil drenching. The rice seedlings were allowed to stand for 3 days. Second instar larvae were released, and the rice seedlings were then allowed to stand in a humidistat chamber (light period 16 hr - dark period 8 hr) (25°C). Three days after the release, the larvae were observed for survival or death, and the death rate of larvae was calculated in the same manner as in Reference Test Example 1. The test was duplicated. As is apparent from Table 17 below, 16-keto aspergillimide exhibited systemic insecticidal activity against Nilaparvata lugens.

Reference Test Example 4: Insecticidal effect against Sogatella furcifera

The death rate against larvae at the second instar born of Sogatella furcifera was calculated in the same manner as in Reference Test Example 3. As is apparent from Table 17 below, 16-keto aspergillimide exhibited systemic insecticidal activity against Sogatella furcifera.

Reference Test Example 5: Insecticidal effect against Neahotettix cincticeps

The death rate against second instar larvae was calculated in the same manner as in Reference Test Example 3. As is apparent from Table 17 below, 16-keto aspergillimide exhibited systemic insecticidal activity against Nephotettix cincticeps.

Reference Test Example 6: Insecticidal effect against Frankliniella occidental is

A test solution adjusted to a predetermined concentration (10% aqueous acetone solution) was applied to cucumber seedlings in pot couture by soil drenching. The cucumber seedlings were allowed to stand for 4 days. A leaf disk having a diameter of 2.8 cm was then cut out from cucumber seedlings, and first instar larvae were released. The leaf disks were then allowed to stand in a humidistat chamber (light period 16 hr - dark period 8 hr) (25°C). Six days after the release, the larvae were observed for survival or death, and the death rate of larvae was calculated in the same manner as in Reference Test Example 1. The test was duplicated. As is apparent from Table 17 below, 16-keto aspergillimide exhibited systemic insecticidal activity against Frankliniella occidentalis.

<Application test of single agent of 16-keto aspergillimide for control of defoliator>

Reference Test Example 7: Insecticidal effect against Sogatella furcifera

A test solution adjusted to a predetermined concentration (10% aqueous acetone solution) was applied to rice seedlings in pot culture. The rice seedlings were air-dried, and second instar larvae were released, and the rice seedlings were then allowed to stand in a humidistat chamber (light period 16 hr - dark period 8 hr) (25°C). Three days after the release, the larvae were observed for survival or death, and the death rate of larvae was calculated in the same manner as in Reference Test Example 1. The test was duplicated. As is apparent from Table 18 below, 16-keto aspergillimide exhibited insecticidal activity against Sogatella furcifera also by foliage application.

Reference Test Example 8: Insecticidal effect against Nephotettix cincticeps

The death rate against larvae at the second instar born of Nephotettix cincticeps was calculated in the same manner as in Reference Test Example 7. As is apparent from Table 18 below, 16-keto aspergillimide exhibited insecticidal activity against Nephotettix cincticeps also in foliage application.

Reference Test Example 9: Insecticidal effect against Frankliniella occidentalis

A leaf disk having a diameter of 2.8 cm was cut out from haricot in pot culture, and a test solution adjusted to a predetermined concentration (50% aqueous acetone solution, with 0.05% Tween 20 added) was applied thereto. The leaf disk was air-air dried, and first instar larvae were released. Thereafter, the leaf disk was then allowed to stand in a humidistat chamber (light period 16 hr - dark period 8 hr) (25°C). Three days after the release, the larvae were observed for survival or death, and the death rate of larvae was calculated in the same manner as in Reference Test Example 1. As is apparent from Table 18 below, 16-keto aspergillimide exhibited insecticidal activity against Frankliniella occidentalis also by foliage application.

[Table 16]

Table 16: Insecticidal effect of 16-keto aspergillimide against Trigonotylus caelestialium

| Preparation name | Treatment concentration (μg/seedling) | Death rate (%) |
|---|---|---|
| Trigonotylus caelestialium | 0.5 | 100 |

[Table 17]

Table 17: Insecticidal effect of 16-keto aspergillimide against various pests

| Preparation name | Treatment concentration (mg/seedling) | Death rate (%) |
|---|---|---|
| Sogatella furcifera | 1.0 | 100 |
| Nilaparvata lugens | 1.0 | 100 |
| Nephotettix cincticeps | 1.0 | 100 |
| Frankliniella occidentalis | 2.0 | 100 |

[Table 18]

Table 18: Insecticidal effect of 16-keto aspergillimide against various pests

| Preparation name | Treatment concentration (ppm) | Death rate (%) |
|---|---|---|
| Sogatella furcifera | 100 | 100 |
| Nephotettix cincticeps | 100 | 100 |
| Frankliniella occidentalis | 100 | 100 |

## Claims

1. A composition for use in controlling harmful organisms, comprising as active ingredients at least one of 16-keto aspergillimide, its enantiomers, their mixture, or their acid addition salts and at least one of other harmful organism control agents.

2. The composition according to claim 1, which further comprises an agriculturally and zootechnically acceptable carrier.

3. The composition according to claim 1, wherein the other harmful organism control agents are agricultural and horticultural insecticides and/or fungicides.

4. The composition according to claim 1, wherein the other harmful organism control agents are an agricultural and horticultural insecticides.

5. The composition according to claim 1, wherein the other harmful organism control agents are a parasite control agents for animals.

6. The composition according to claim 1, wherein the other harmful organism control agents are an ectoparasite control agents for animals.

7. The composition according to claim 1, wherein the other harmful organism control agents are selected from the group consisting of organic phosphoric ester compounds, carbamate compounds, nereistoxin derivatives, organochlorine compounds, pyrethroid compounds, benzyl urea compounds, juvenile hormone-like compounds, molting hormone-like compounds, neonicotinoid compounds, sodium channel blockers for nerve cells, insecticidalmacrocyclic lactones, γ-aminobutyric acid (GABA)antagonists, ryanodine receptor agonistic compounds, insecticidal ureas, BT agents, entomopathogenic viral agents, polyether antibiotics, thiamine antagonists and sulfa drugs/folic acid antagonist compounding agents.

8. The composition according to claim 1, wherein the other harmful organism control agents are selected from the group consisting of acephate, dichlorvos, EPN, fenitothion, fenamifos, prothiofos, profenofos, pyraclofos, chlorpyrifos-methyl, haloxon, coumaphos, malathion, dimpylate, naled, tetradifon, diazinon, methomyl, thiodicarb, aldicarb, oxamyl, propoxur, carbaryl, fenobucarb, ethiofencarb, fenothiocarb, pirimicarb, carbofuran, benfuracarb, cartap, thiocyclam, dicofol, tetradifon, permethrin, tefluthrin, cypermethrin, deltamethrin, cyhalothrin, fenvalerate, fluvalinate, ethofenprox, silafluofen, diflubenzuron, teflubenzuron, flufenoxuron, chlorfluazuron, methoprene, chromafenozide, buprofezin, hexythiazox, amitraz, chlordimeform, pyridaben, fenpyroxymate, pyrimidifen, tebufenpyrad, fluacrypyrim, acequinocyl, cyflumetofen, flubendiamide, ethiprole, fipronil, ethoxazole, imidacloprid, clothianidin, thiamethoxam, thiacloprid, sulfoxaflor, pymetrozine, bifenazate, spirodiclofen, spiromesifen, flonicamid, chlorfenapyr, pyriproxyfene, indoxacarb, pyridalyl, spinosad, avermectin, milbemycin, milbemycin oxime, maduramycin, BT agents, entomopathogenic viral agents, emamectinbenzoate, spinetoram, pyrifluquinazon, chlorantraniliprole, cyenopyrafen, spirotetramat, lepimectin, metaflumizone, pyrafluprole, pyriprole, dimefluthrin, fenazaflor, hydramethylnon, triazamate, ivermectin, selamectin, moxidectin, doramectin, eprinomectin, dinotefuran, nitenpyram, acetamiprid, allethrin, d·d-T allethrin, dl·d-T80 allethrin, pyrethrins, phenothrin, flumethrin, cyfluthrin, d·d-T80 prarethrin, phthalthrin, transfluthrine, resmethrin, cyphenothrin, pyrethrum extract, synepirin222, synepirin 500, pyriproxyfene, lufenuron, deet, trichlofon, tetrachlorvinphos, bromofenofos, cythioate, metoxadiazon, cyromazine, triflumuron, star anise oil, triclabendazole, flubendazole, fenbendazole, parbendazole, tiabendazole, antimony sodium gluconate, levamisole hydrochloride, bithionol, dichlorofen, bromopropylate, piperonylbutoxide, phenothiazine, piperazine carbon bisulfide, piperazine phosphate, piperazine adipate, piperazine citrate, melarsomine dihydrochloride, metyridine, santonin, pyrantel pamoate, pyrantel, morantel, praziquantel, febantel, emodepside, hygtomycin B, destomycin A, clorsulon, nitroxynil, diamfenethide, antimony sodium gluconate, levamisole hydrochloride, melrsonyl, dithiazanine iodide, stibophen, disophenol, dietylcarbamazine, diminazene, acrinamine, metronidazole, santonin, bunamidine, arecoline and compounds represented by formula (I) or agriculturally and horticulturally acceptable acid addition salts thereof:

[Chemical formula 1]

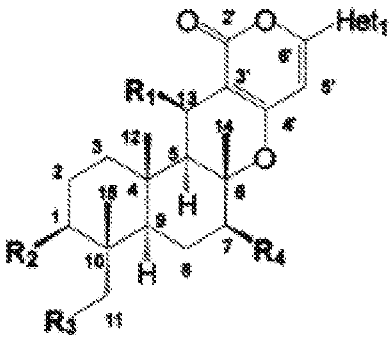

(I)

wherein $Het_1$ represents 3-pyridyl; $R_1$ represents hydroxyl; $R_2$ and $R_3$ represent cyclopropylcarbonyloxy; and $R_4$ represents a hydrogen atom or hydroxyl.

**9.** The composition according to claim 1, wherein the other harmful organism control agents are selected from the group consisting of imidacloprid, nitenpyram, clothianidin, acetamiprid, dinotefuran, thiacloprid, thiamethoxam, sulfoxaflor, fipronil, ivermectin and compounds represented by formula (I) according to claim 8.

**10.** A combination comprising as active ingredients at least one of 16-keto aspergillimide, its enantiomers, their mixture, or their acid addition salts and at least one of other harmful organism control agents.

**11.** A method for protecting useful plants or animals from harmful organisms, the method comprising applying at least one of 16-keto aspergillimide, its enantiomers, their mixture, or their acid addition salts and at least one of other harmful organism control agents simultaneously or separately from each other to an area to be treated.

**12.** The method according to claim 11, wherein the ingredients are simultaneously applied to the area to be treated.

**13.** A method for protecting useful plants or animals from harmful organisms, comprising applying an effective amount of a compositon for use in controlling harmful organisms according to claims 1 to 9 to an object, wherein the object isa harmful organism, a useful plant, a seed of a useful plant, a soil, a cultivation plant, or an animal.

**14.** The method according to claim 13, wherein the object is a useful plant.

**15.** The method according to claim 13, wherein the object is a soil.

**16.** The method according to claim 13, wherein the object is an animal.

**17.** A method for protecting useful plants or animals from harmful organisms, comprising applying an effective amount of a combination according to claim 10 to an object harmful organism, an object useful plant, a seed of an object useful plant, a soil, a cultivation plant, or an object animal.

**18.** Use of a composition for use in controlling harmful organisms according to claims 1 to 9 for the protection of useful plants or animals from harmful organisms.

**19.** Use of a combination according to claim 10 for the protection of useful plants or animals from harmful organisms.

**INTERNATIONAL SEARCH REPORT**

International application No. PCT/JP2009/071255

A. CLASSIFICATION OF SUBJECT MATTER
*A01N43/90*(2006.01)i, *A01N43/40*(2006.01)i, *A01N47/02*(2006.01)i, *A01N47/40*(2006.01)i, *A01N51/00*(2006.01)i, *A01P3/00*(2006.01)i, *A01P7/02*(2006.01)i, *A01P7/04*(2006.01)i

According to International Patent Classification (IPC) or to both national classification and IPC

B. FIELDS SEARCHED

Minimum documentation searched (classification system followed by classification symbols)
A01N43/90, A01N43/40, A01N47/02, A01N47/40, A01N51/00, A01P3/00, A01P7/02, A01P7/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2008/156165 A1 (Meiji Seika Kaisha, Ltd.), 24 December 2008 (24.12.2008), claims; paragraphs [0041] to [0043]; examples & JP 4234196 B | 1-19 |
| A | JP 10-245383 A (Hideo HAYASHI), 14 September 1998 (14.09.1998), claims (Family: none) | 1-19 |
| A | Journal of Antibiotics, 1997, 50(10), p.840-846 | 1-19 |
| A | Journal of the American Chemical Society, 2003, 125(48), p.14692-14693 | 1-19 |
| A | Bioscience, Biotechnology, and Biochemistry, 2000, 64(1), p.111-115 | 1-19 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 January, 2010 (08.01.10) | 02 February, 2010 (02.02.10) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/071255

C (Continuation). DOCUMENTS CONSIDERED TO BE RELEVANT

| Category* | Citation of document, with indication, where appropriate, of the relevant passages | Relevant to claim No. |
|---|---|---|
| A | Tetrahedron Letters, 1997, 38(32), p.5655-5658 | 1-19 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008323739 A
- JP 10245383 A
- JP 4015182 B


### Non-patent literature cited in the description


- **Kyohei Yamashita et al.** Noyaku no Kagaku (Agrochemical Science). Buneido Publishing Co., Ltd, 14
- *The Journal of Antibiotics,* vol. 50 (10), 840-846
- **Further.** *Tetrahedron Letters,* 1997, vol. 38 (32), 5655-5658
- *Bioscience, Biotechnology and Biochemistry,* 2000, vol. 64 (1), 111-115
- *The Journal of Antibiotics,* 1997, vol. 50 (10), 840-846
- *Tetrahedron Letters,* 1997, vol. 38 (32), 5655-5658
- The Pesticide Manual. The British Crop Protection Council
- SHIBUYA INDEX. SHIBUYA INDEX RESEARCH GROUP. 2008
- pesticide Science Society of Japan/Seyoho Kenkyukai (Special Committee on Agricultural Formulation and Application). Noyaku Seizai Gaido (Guide for Pesticide Formulation). Japan Plant Protection Association, 1997